# EUROPEAN PATENT APPLICATION

(11) **EP 3 919 124 A1**
(43) Date of publication of application: **08.12.2021**
(21) Application number: 20747635.9
(22) Date of filing: 31.01.2020
(51) Int. Cl.: A61P 1/04, A61P 11/02, A61P 11/06, A61P 19/02, A61P 21/00, A61P 25/00, A61P 25/02, A61P 25/16, A61P 25/26, A61P 27/14, A61P 29/00, A61P 37/02, A61P 37/06, A61P 37/08, A61P 43/00, A61P 17/00, A61P 17/04, A61P 17/06, C07K 16/28, A61K 35/15, A61K 35/545

(54) **METHOD FOR DETECTING CELLS**

(30) Priority: 01.02.2019 JP 2019017293
(71) Applicant: Kyoto University, Kyoto-shi, Kyoto 606-8501 (JP); Takeda Pharmaceutical Company Limited, Osaka-shi, Osaka 541-0045 (JP)
(72) Inventor: KANEKO, Shin, Kyoto-shi, Kyoto 606-8501 (JP); IRIGUCHI, Shoichi, Kyoto-shi, Kyoto 606-8501 (JP); SEKIYA, Keiko, Fujisawa-shi, Kanagawa 251-0012 (JP); KASSAI, Yoshiaki, Fujisawa-shi, Kanagawa 251-0012 (JP)
(74) Representative: Jones, Nicholas Andrew
(86) International application number: PCT/JP2020/003628
(87) International publication number: WO 2020/158914

(57) **Abstract**

Disclosed is a method for detecting regulatory dendritic cells, the method comprising step a: detecting the presence or absence of one or more types of molecules on a cell surface in a cell population that comprises regulatory dendritic cells; and step b: identifying regulatory dendritic cells on the basis of the presence or absence of the one or more types of molecules. Also disclosed is a method for producing a cell population enriched for regulatory dendritic cells, the method comprising step 1: detecting the presence or absence of one or more types of molecules on a cell surface in a cell population that comprises regulatory dendritic cells; and step 2: obtaining a cell population enriched for regulatory dendritic cells on the basis of the presence or absence of the one or more types of molecules. Further disclosed is a cell population enriched for regulatory dendritic cells obtained by the method.

## Description

### Technical Field

The present invention relates to a method for detecting regulatory dendritic cells; a method for producing a cell population enriched for regulatory dendritic cells; a method for enriching regulatory dendritic cells; a cell population enriched for regulatory dendritic cells; a reagent for detecting regulatory dendritic cells; and a pharmaceutical composition comprising the cell population enriched for regulatory dendritic cells.

### Background of Invention

Dendritic cells, which have dendrites, act as antigen-presenting cells at the initiation of an immune response expressing major histocompatibility complex (MHC) class II molecules. Dendritic cells differentiate from hematopoietic stem cells to immature dendritic cells through the myeloid differentiation pathway, and then to mature dendritic cells.

In peripheral inflammatory tissues, immature dendritic cells phagocytize foreign antigens such as microorganisms, viruses, and foreign substances that have invaded the peripheral inflammatory tissues; differentiate into mature dendritic cells; and migrate to the secondary lymphoid tissues to which they belong. Mature dendritic cells provide antigen stimulation and co-stimulation to naive T cells to induce differentiation of antigen-specific effector T cells and elicit a primary immune response. (These dendritic cells (conventional dendritic cells) are sometimes referred to below as "conDCs.")

On the other hand, some dendritic cells express fewer co-stimulatory molecules even under inflammatory conditions; this suggests that these dendritic cells regulate T cells in a suppressive manner and induce immune tolerance to self-tissues etc. These dendritic cells are called regulatory dendritic cells (sometimes referred to below as "regDCs").

As stated above, regulatory dendritic cells have been suggested to induce immune tolerance. In view of this, if regulatory dendritic cells can be replenished or regenerated, a wide range of clinical applications to, for example, immunosuppression during organ transplantation (e.g., achieving immunosuppressant-free transplantation of organs) and autoimmune diseases (e.g., inflammatory bowel diseases) can be expected. Given this, attempts have been made, for example, to induce hematopoietic progenitor cells (sometimes referred to below as "HPCs") from iPS cells, then induce myeloid progenitor cells (i.e., myeloid progenitor, sometimes referred to below as "MP") from the HPCs, and then induce dendritic cells from the MP.

For example, Non-patent Literature (NPL) 1 discloses induction of monocyte-derived immature dendritic cells by culturing rhesus macaque CD14⁺ monocytes in a medium supplemented with GM-CSF, IL-4, vitamin D3, and IL-10. NPL 2 discloses induction of dendritic cells from human iPS cells, and the establishment of a xeno-free culture protocol to induce dendritic cells from iPS cells. Further, NPL 3 discloses induction of regulatory dendritic cells from murine induced iPS cells, and discloses that the obtained regulatory dendritic cells showed immune responsiveness regulation effects both *in vitro* and *in vivo,* and the ability to generate regulatory T-cells *in vitro.*

### Citation List

### Non-patent Literature

NPL 1: AF Zahorchak et al., Transplantation, 84 (2007); 196-206
NPL 2: S Senju et al., Gene Therapy, 18 (2011), 874-883
NPL 3: Q Zhang et al., Scientific Reports, 4:3979, DOI:10.1038/srep03979

### Summary of Invention

### Technical Problem

As described above, methods for inducing regulatory dendritic cells from rhesus macaque monocytes, mouse iPS cells, and the like have been reported. However, cell groups obtained by these methods also include conDCs. For clinical applications etc., it is desirable to use purified regulatory dendritic cells. However, no markers specific to regulatory dendritic cells have been known so far, and methods for identifying and separating regulatory dendritic cells have not yet been established.

An object of the present invention is to provide a method for detecting regulatory dendritic cells; a method for producing a cell population enriched for regulatory dendritic cells; a method for enriching regulatory dendritic cells; a cell population enriched for regulatory dendritic cells; a reagent for detecting regulatory dendritic cells; and a pharmaceutical composition comprising the cell population enriched for regulatory dendritic cells.

### Solution to Problem

The present inventors conducted extensive research to solve the above problems, and consequently found that regulatory dendritic cells can be identified and separated on the basis of the results of detection of the presence or absence of specific molecules on a cell surface in a cell population that comprises regulatory dendritic cells.

Based on these findings, further research has been conducted to thus complete the present invention. More specifically, the present invention provides the following method for detecting regulatory dendritic cells; method for producing a cell population enriched for regulatory dendritic cells; method for enriching regulatory dendritic cells; cell population enriched for regulatory dendritic cells; reagent for detecting regulatory dendritic cells; and pharmaceutical composition.

[1] A method for detecting regulatory dendritic cells, the method comprising:
   step a: detecting the presence or absence of one or more types of molecules on a cell surface in a cell population that comprises regulatory dendritic cells; and
   step b: identifying regulatory dendritic cells on the basis of the presence or absence of the one or more types of molecules.
[2] The method according to [1], wherein the one or more types of molecules are selected from the group consisting of CD99, CD183, CD107a, CD366, CD172a, CD200R, CD44, CD206, CD181, CD354, CD300c, CD50, and CD84.
[3] The method according to [1], wherein the one or more types of molecules are two or more types of molecules selected from the group consisting of CD99, CD183, CD107a, CD366, CD172a, CD200R, CD44, CD206, CD181, CD354, CD300c, CD50, and CD84.
[3-1] The method according to [1], wherein the one or more types of molecules are CD200R and CD44.
[4] The method according to any one of [1] to [3-1], wherein the cell population that comprises regulatory dendritic cells is a cell population obtained by inducing differentiation of pluripotent stem cells.
[5] The method according to [4], wherein the pluripotent stem cells are human-induced pluripotent stem cells.
[6] A method for producing a cell population enriched for regulatory dendritic cells, the method comprising:
   step 1: detecting the presence or absence of one or more types of molecules on a cell surface in a cell population that comprises regulatory dendritic cells; and
   step 2: obtaining a cell population enriched for regulatory dendritic cells on the basis of the presence or absence of the one or more types of molecules.
[7] The method according to [6], wherein the one or more types of molecules are selected from the group consisting of CD99, CD183, CD107a, CD366, CD172a, CD200R, CD44, CD206, CD181, CD354, CD300c, CD50, and CD84.
[8] The method according to [6], wherein the one or more types of molecules are two or more types of molecules selected from the group consisting of CD99, CD183, CD107a, CD366, CD172a, CD200R, CD44, CD206, CD181, CD354, CD300c, CD50, and CD84.
[8-1] The method according to [6], wherein the one or more types of molecules are CD200R and CD44.
[9] A method for enriching regulatory dendritic cells, the method comprising:
   step A: detecting the presence or absence of one or more types of molecules on a cell surface in a cell population that comprises regulatory dendritic cells; and
   step B: selecting regulatory dendritic cells on the basis of the presence or absence of the one or more types of molecules.
[9-1] The method according to [9], wherein the one or more types of molecules are selected from the group consisting of CD99, CD183, CD107a, CD366, CD172a, CD200R, CD44, CD206, CD181, CD354, CD300c, CD50, and CD84.
[9-2] The method according to [9], wherein the one or more types of molecules are two or more types of molecules selected from the group consisting of CD99, CD183, CD107a, CD366, CD172a, CD200R, CD44, CD206, CD181, CD354, CD300c, CD50, and CD84.
[9-3] The method according to [9], wherein the one or more types of molecules are CD200R and CD44.
[10] A cell population enriched for regulatory dendritic cells obtained by the method of any one of [6] to [9-3].
[11] A cell population enriched for regulatory dendritic cells that express or do not express one or more types of molecules selected from the group consisting of CD99, CD183, CD107a, CD366, CD172a, CD200R, CD44, CD206, CD181, CD354, CD300c, CD50, and CD84.
[12] A cell population enriched for regulatory dendritic cells that express or do not express two or more types of molecules selected from the group consisting of CD99, CD183, CD107a, CD366, CD172a, CD200R, CD44, CD206, CD181, CD354, CD300c, CD50, and CD84.
[12-1] A cell population enriched for regulatory dendritic cells that express or do not express CD200R and CD44.
[13] A reagent for detecting regulatory dendritic cells, the reagent comprising one or more antibodies that are each against each of one or more types of molecules selected from the group consisting of CD99, CD183, CD107a, CD366, CD172a, CD200R, CD44, CD206, CD181, CD354, CD300c, CD50, and CD84.
[13-1] A reagent for detecting regulatory dendritic cells, the reagent comprising two or more antibodies that are each against each of two or more types of molecules selected from the group consisting of CD99, CD183, CD107a, CD366, CD172a, CD200R, CD44, CD206, CD181, CD354, CD300c, CD50, and CD84.
[13-2] A reagent for detecting regulatory dendritic cells, the reagent comprising antibodies that are each against CD200R or CD44.
[14] A pharmaceutical composition comprising the cell population enriched for regulatory dendritic cells of any one of [10] to [12-1].

### Advantageous Effects of Invention

The present invention enables the detection of regulatory dendritic cells in a cell population that comprises regulatory dendritic cells, and the enrichment of regulatory dendritic cells in a cell population that comprises regulatory dendritic cells.

### Description of Embodiments

Embodiments of the present invention are described in detail below.

In this specification, the term "comprise" also encompasses the meanings of both "essentially consisting of" and "consisting of."

The method for detecting regulatory dendritic cells according to the present invention is characterized by comprising the following steps:
step a: detecting the presence or absence of one or more types of molecules on a cell surface in a cell population that comprises regulatory dendritic cells; and
step b: identifying regulatory dendritic cells on the basis of the presence or absence of the one or more types of molecules.

The method for producing a cell population enriched for regulatory dendritic cells according to the present invention is characterized by comprising the following steps:
step 1: detecting the presence or absence of one or more types of molecules on a cell surface in a cell population that comprises regulatory dendritic cells; and
step 2: obtaining a cell population enriched for regulatory dendritic cells on the basis of the presence or absence of the one or more types of molecules.

The method for enriching regulatory dendritic cells according to the present invention is characterized by comprising the following steps:
step A: detecting the presence or absence of one or more types of molecules on a cell surface in a cell population that comprises regulatory dendritic cells; and
step B: selecting regulatory dendritic cells on the basis of the presence or absence of the one or more types of molecules.

In this specification, the term "cell population" means one or more cells of the same type or different types. The term "cell population" also means a mass of cells of the same or different types.

In this specification, the terms "enrich" and "enrichment" refer to an increase in the amount of a specific constituent component in a composition such as a cell composition. When a cell composition, e.g., a cell population, is described by using the term "enriched," it means that the amount of a specific constituent component is increased in the cell population compared to the percentage of the constituent component in the cell population before being enriched. For example, a composition such as a cell population can be enriched in terms of a target cell type, so that the percentage of the target cell type increases compared to the percentage of the target cells present in the cell population before being enriched. In a specific embodiment according to the present invention, a cell population is enriched in terms of a target cell population by at least 1%, 2%, 3%, 4%, 5%, 6%, 7%, 8%, 9%, 10%, 20%, 30%, 40%, 50%, 60%, 70%, 80%, 90%, or 95% (i.e. the percentage of the target cell population increases) by a method for enriching the target cell population. In a specific embodiment according to the present invention, the cell population enriched by the method for enriching the target cell population comprises at least 1%, 2%, 3%, 4%, 5%, 6%, 7%, 8%, 9%, 10%, 20%, 30%, 40%, 50%, 60%, 70%, 80% or 90%, or 100% of the target cell population.

In this specification, the expression "the presence or absence of one or more types of molecules on a cell surface" means that the presence or absence is determined for each molecule when two or more types of molecules are concerned (for example, one molecule is determined to be present whereas another molecule is determined to be absent). (Similar expressions have the same meaning.)

In this specification, the term "pluripotent stem cells" refers to embryonic stem cells (ES cells) and cells with pluripotency similar to that of ES cells, i.e., cells that have the potential to differentiate into various biological tissues (including all of endoderm, mesoderm, and ectoderm). Examples of cells with pluripotency similar to that of ES cells include induced pluripotent stem cells (sometimes referred to as "iPS cells" in this specification).

In the present invention, the term "regulatory dendritic cells" means dendritic cells that express fewer co-stimulatory molecules even under inflammatory conditions, and that regulate T cells in a suppressive manner and induce immune tolerance to self-tissues etc. Regulatory dendritic cells preferably refer to cells that exhibit an ability to produce interleukin 10 (IL-10), among dendritic cells that express CD11b and CD11c (CD11b/c double positive cells). The cells that exhibit an ability to produce IL-10 refer to cells that substantially produce IL-10. The cell production of IL-10 can be detected by flow cytometry and other known methods.

The regulatory dendritic cells for use in the present invention may be any regulatory dendritic cells. For example, the regulatory dendritic cells may be regulatory dendritic cells induced *in vitro* according to known methods (e.g., the methods disclosed in NPL 1 and NPL 3) (e.g., regulatory dendritic cells obtained by inducing differentiation of pluripotent stem cells), or regulatory dendritic cells isolated from biological tissue by known methods.

Examples of pluripotent stem cells include embryonic stem cells (ES cells), induced pluripotent stem cells (iPS cells), embryonic carcinoma cells (EC cells), and embryonic germ stem cells (EG cells). The pluripotent stem cells are preferably iPS cells (more preferably human iPS cells). When the pluripotent stem cells are ES cells or any cells derived from human embryo, those cells may be produced by destroying the embryo or without destroying the embryo, and are preferably produced without destroying the embryo.

For "ES cells," various mouse ES cell lines established by inGenious Targeting Laboratory, RIKEN (Institute of Physical and Chemical Research), and the like are available as mouse ES cells; and various human ES cell lines established by the University of Wisconsin, NIH, RIKEN, Kyoto University, the National Center for Child Health and Development, Cellartis, and the like are available as human ES cells. For example, human ES cell lines for use may be CHB-1 to CHB-12, RUES1, RUES2, HUES1 to HUES28, and other cell lines distributed by ESI Bio; H1, H9, and other cell lines distributed by WiCell Research Institute; and KhES-1, KhES-2, KhES-3, KhES-4, KhES-5, SSES1, SSES2, SSES3, and other cell lines distributed by RIKEN.

The term "induced pluripotent stem cells" refers to cells that are obtained by reprogramming mammalian somatic cells or undifferentiated stem cells by introducing specific factors (nuclear reprogramming factors). Various induced pluripotent stem cells are available at present. Usable induced pluripotent stem cells include iPS cells established by Yamanaka et al. by introducing four factors, Oct3/4, Sox2, Klf4, and c-Myc, into mouse fibroblasts (Takahashi K, Yamanaka S., Cell, (2006) 126: 663-676); iPS cells derived from human cells, established by introducing similar four factors into human fibroblasts (Takahashi K, Yamanaka S., et al. Cell, (2007) 131: 861-872); Nanog-iPS cells established by sorting cells using expression of Nanog as an indicator after introduction of the 4 factors (Okita, K., Ichisaka, T., and Yamanaka, S. (2007), Nature 448, 313-317); iPS cells produced by a method not using c-Myc (Nakagawa M, Yamanaka S., et al., Nature Biotechnology, (2008) 26, 101-106); and iPS cells established by introducing 6 factors by a virus-free method (Okita K et al. Nat. Methods 2011 May; 8(5): 409-12, Okita K et al. Stem Cells. 31(3): 458-66). It is also possible to use induced pluripotent stem cells established by Thomson et al. by introducing 4 factors OCT3/4, SOX2, NANOG, and LIN28 (Yu J., Thomson JA. et al., Science (2007) 318: 1917-1920); induced pluripotent stem cells produced by Daley et al. (Park IH, Daley GQ. et al., Nature (2007) 451: 141-146); induced pluripotent stem cells produced by Sakurada et al. (JP2008-307007A); and the like.

In addition, usable induced pluripotent stem cells also include any induced pluripotent stem cells known in the related fields and disclosed in all published articles (e.g., Shi Y., Ding S., et al., Cell Stem Cell, (2008) Vol. 3, Issue 5, 568-574; Kim JB., Scholer HR., et al., Nature, (2008) 454, 646-650; Huangfu D., Melton, DA., et al., Nature Biotechnology, (2008) 26, No. 7, 795-797), as well as patent documents (e.g., JP2008-307007A, JP2008-283972A, US Patent Publication No. 2008/2336610, US Patent Publication No. 2009/047263, WO2007/069666, WO2008/118220, WO2008/124133, WO2008/151058, WO2009/006930, WO2009/006997, and WO 2009/007852).

Available induced pluripotent cell lines include various iPS cell lines established by NIH, RIKEN, Kyoto University, and the like. Examples of human iPS cell lines include HiPS-RIKEN-1A, HiPS-RIKEN-2A, HiPS-RIKEN-12A, and Nips-B2 from RIKEN; and 253G1, 201B7, 409B2, 454E2, 606A1, 610B1, and 648A1 from Kyoto University.

Regulatory dendritic cells can be produced by subjecting pluripotent stem cells to known methods. When the pluripotent stem cells are iPS cells, hematopoietic progenitor cells can be produced, for example, by the method disclosed in WO2017/221975 and the method disclosed in Cell Reports 2 (2012), 1722-1735. Subsequently, myeloid progenitor cells are induced from the hematopoietic progenitor cells, for example, by using the methods disclosed in NPL 1 to NPL 3; and then regulatory dendritic cells can be produced by inducing dendritic cells from the myeloid progenitor cells.

The biological tissues from which regulatory dendritic cells are isolated may be any biological tissues that contain regulatory dendritic cells. Examples include lymph nodes, thymus, spleen, intestinal tract, and skin.

The regulatory dendritic cells and the pluripotent stem cells for use in the present invention are preferably cells that meet good manufacturing practice (GMP) standards, from the standpoint of therapeutic application.

In this specification, the molecules on cell surfaces that enable detection, enrichment, and/or separation of regulatory dendritic cells are referred to as "regDC markers."

The regDC marker is preferably at least one molecule selected from the group consisting of CD99, CD183 (CXCR3), CD107a, CD366 (Tim3), CD172a (SIRPa), CD200R, CD44, CD206 (MR), CD181 (CXCR1), CD354 (TREM1), CD300c, CD50, and CD84. Examples of combinations of the regDC markers include any combinations of two, three, four, five, six, seven, eight, nine, ten, eleven, twelve, or thirteen types of molecules. In particular, combinations of two or more types of molecules selected from the group consisting of CD99, CD183, CD107a, CD366, CD172a, CD200R, CD44, CD206, CD181, CD354, CD300c, CD50, and CD84 are preferred.

In this specification, a marker whose presence on a cell surface is used as an index may be referred to as a "regDC positive marker," whereas a marker whose absence on a cell surface is used as an index may be referred to as a "regDC negative marker." In the present invention, cells in which the regDC positive marker is present may be referred to as "regDC marker positive cells." For example, cells that express the CD200R molecule may be referred to as CD200R positive cells. Further, in the present invention, cells in which the regDC negative marker is not present may be referred to as "regDC marker negative cells." For example, cells that do not express the CD44 molecule may be referred to as "CD44 negative cells."

In the present invention, each regDC marker is not limited only for use as either a regDC positive marker or a regDC negative marker. If the regDC marker can be used as both a regDC positive marker and a regDC negative marker, for example, one regDC marker can be used as a regDC positive marker for the detection of regulatory dendritic cells, and also used as a regDC negative marker for the enrichment of regulatory dendritic cells. When two different types of regDC markers are combined for detection and enrichment, there will be four different combinations of the regDC positive markers and the regDC negative markers. Of these, two or more combinations can sometimes be used for the detection and enrichment of regulatory dendritic cells. (For example, there are four different combinations for regDC markers A and B, which are combinations of A⁺B⁺, A⁻B⁻, A⁺B⁻, and A⁻B⁺; of these, two or more of these combinations (e.g., A⁺B⁻ and A⁺B⁻) can sometimes be used.) It is also possible to use different combinations for detection and enrichment, respectively. This also applies to cases where a combination of three or more types of regDC markers is used.

Specific examples of combinations of two types of molecules of regDC markers include CD99 and CD183, CD99 and CD107a, CD99 and CD366, CD99 and CD172a, CD99 and CD200R, CD99 and CD44, CD99 and CD206, CD99 and CD181, CD99 and CD354, CD99 and CD300c, CD99 and CD50, CD99 and CD84, CD183 and CD107a, CD183 and CD366, CD183 and CD172a, CD183 and CD200R, CD183 and CD44, CD183 and CD206, CD183 and CD181, CD183 and CD354, CD183 and CD300c, CD183 and CD50, CD183 and CD84, CD107a and CD366, CD107a and CD172a, CD107a and CD200R, CD107a and CD44, CD107a and CD206, CD107a and CD181, CD107a and CD354, CD107a and CD300c, CD107a and CD50, CD107a and CD84, CD366 and CD172a, CD366 and CD200R, CD366 and CD44, CD366 and CD206, CD366 and CD181, CD366 and CD354, CD366 and CD300c, CD366 and CD50, CD366 and CD84, CD172a and CD200R, CD172a and CD44, CD172a and CD206, CD172a and CD181, CD172a and CD354, CD172a and CD300c, CD172a and CD50, CD172a and CD84, CD200R and CD44, CD200R and CD206, CD200R and CD181, CD200R and CD354, CD200R and CD300c, CD200R and CD50, CD200R and CD84, CD44 and CD206, CD44 and CD181, CD44 and CD354, CD44 and CD300c, CD44 and CD50, CD44 and CD84, CD206 and CD181, CD206 and CD354, CD206 and CD300c, CD206 and CD50, CD206 and CD84, CD181 and CD354, CD181 and CD300c, CD181 and CD50, CD181 and CD84, CD354 and CD300c, CD354 and CD50, CD354 and CD84, CD300c and CD50, CD300c and CD84, and CD50 and CD84.

Of these, examples of preferable combinations of two types of molecules of regDC markers include CD99 and CD183, CD99 and CD107a, CD99 and CD366, CD99 and CD200R, CD99 and CD44, CD99 and CD300c, CD99 and CD50, CD99 and CD84, CD183 and CD107a, CD183 and CD366, CD183 and CD44, CD107a and CD366, CD107a and CD200R, CD172a and CD206, CD172a and CD200R, CD172a and CD44, CD200R and CD44, CD200R and CD206, CD44 and CD206, CD44 and CD300c, CD44 and CD50, CD44 and CD84, CD181 and CD354, CD300c and CD50, and CD50 and CD84.

For the enrichment of regulatory dendritic cells, examples of preferable combinations of two types of molecules of regDC markers include CD99 and CD183 (N (negative):P (positive), P:P, P:N), CD99 and CD107a (P:P, P:N, N:N), CD99 and CD366 (N:N), CD99 and CD200R (N:P, P:P), CD99 and CD44 (N:P, P:P, P:N), CD99 and CD300c (P:P, P:N, N:N), CD99 and CD50 (N:P, P:P, P:N), CD99 and CD84 (N:P, P:P), CD183 and CD107a (P:N, N:N), CD183 and CD366 (P:P, P:N, N:N), CD183 and CD44 (N:P, P:P), CD107a and CD366 (N:P, N:N), CD107a and CD200R (N:P, P:P), CD172a and CD206 (N:N), CD172a and CD200R (N:P, P:P), CD172a and CD44 (N:N), CD200R and CD44 (P:P, P:N), CD200R and CD206 (P:P, P:N), CD44 and CD206 (N:N), CD44 and CD300c (P:P, P:N, N:N), CD44 and CD50 (N:P, P:P, P:N), CD44 and CD84 (P:P, N:P), CD181 and CD354 (N:P, P:P), CD300c and CD50 (N:P, P:P, N:N), and CD50 and CD84 (P:N, P:P). In the parentheses above, preferred combinations of positive (P) and negative (N) markers are shown (the same applies below). For example, the expression "CD99 and CD183 (N:P)" indicates that CD99 is preferably used as a regDC negative marker, while CD183 is preferably used as a regDC positive marker.

The combination of two types of molecules of regDC markers is more preferably, for example, a combination of CD99 and CD107a (N:N), CD99 and CD200R (P:P), CD99 and CD44 (P:P, P:N), CD99 and CD50 (N:P, P:P), CD183 and CD107a (P:N), CD107a and CD366 (N:P), CD172a and CD200R (N:P, P:P), CD200R and CD44 (P:N), CD200R and CD206 (P:N), CD44 and CD50 (N:P, P:P), CD44 and CD84 (N:P), CD300c and CD50 (N:P, P:P), or CD50 and CD84 (P:N, P:P) .

The combination of two types of molecules of regDC markers is still more preferably, for example, a combination of CD99 and CD50 (N:P, P:P), CD172a and CD200R (N:P), CD200R and CD44 (P:N), CD200R and CD206 (P:N), CD44 and CD50 (N:P, P:P), CD44 and CD84 (N:P), CD300c and CD50 (N:P, P:P), or CD50 and CD84 (P:N, P:P).

The combination of two types of molecules of regDC markers is even more preferably, for example, a combination of CD99 and CD50 (N:P, P:P), CD200R and CD44 (P:N), CD200R and CD206 (P:N), CD44 and CD50 (N:P, P:P), CD300c and CD50 (N:P, P:P), or CD50 and CD84 (P:P).

The combination of two types of molecules of regDC markers is still even more preferably, for example, a combination of CD200R and CD44 (P:N).

For the detection of regulatory dendritic cells, examples of preferable combinations of two types of molecules of regDC markers include combinations of CD99 and CD183 (N:P, P:P, P:N, N:N), CD99 and CD107a (N:P, P:P, P:N, N:N), CD99 and CD366 (N:P, P:P, P:N, N:N), CD99 and CD200R (N:P, P:P, N:N), CD99 and CD44 (N:P, P:P, P:N, N:N), CD99 and CD300c (N:P, P:P, P:N, N:N), CD99 and CD50 (N:P, P:P, P:N, N:N), CD99 and CD84 (N:P, P:P, N:N), CD183 and CD107a (N:P, P:P, P:N, N:N), CD183 and CD366 (N:P, P:P, P:N, N:N), CD183 and CD44 (N:P, P:P, P:N, N:N), CD107a and CD366 (N:P, P:P, N:N), CD107a and CD200R (N:P, P:P, P:N, N:N), CD172a and CD206 (N:P, P:N, N:N), CD172a and CD200R (N:P, P:P, N:N), CD172a and CD44 (N:P, P:N, N:N), CD200R and CD44 (P:P, P:N, N:N), CD200R and CD206 (P:P, P:N), CD44 and CD206 (N:P, P:P, N:N), CD44 and CD300c (N:P, P:P, P:N, N:N), CD44 and CD50 (N:P, P:P, P:N, N:N), CD44 and CD84 (N:P, P:P, N:N), CD181 and CD354 (N:P, P:P, P:N, N:N), CD300c and CD50 (N:P, P:P, P:N, N:N), and CD50 and CD84 (N:P, P:P, P:N, N:N).

The combination of two types of molecules of regDC markers is more preferably, for example, a combination of CD99 and CD183 (N:P), CD99 and CD107a (N:N), CD99 and CD366 (N:P), CD99 and CD200R (N:P), CD99 and CD44 (N:N), CD99 and CD300c (N:N), CD99 and CD50 (N:P), CD99 and CD84 (N:N), CD183 and CD107a (P:N), CD183 and CD44 (P:N), CD172a and CD206 (N:N), CD172a and CD200R (N:P), CD172a and CD44 (N:N), CD200R and CD44 (P:N), CD200R and CD206 (P:N), CD44 and CD206 (N:N), CD44 and CD50 (N:P), CD44 and CD84 (N:P), CD181 and CD354 (N:P), CD300c and CD50 (N:P), or CD50 and CD84 (P:N).

The combination of two types of molecules of regDC markers is still more preferably, for example, a combination of CD99 and CD183 (N:P), CD99 and CD107a (N:N), CD99 and CD200R (N:P), CD99 and CD44 (N:N), CD99 and CD50 (N:P), CD183 and CD107a (P:N), CD183 and CD44 (P:N), CD172a and CD206 (N:N), CD172a and CD200R (N:P), CD172a and CD44 (N:N), CD200R and CD44 (P:N), CD200R and CD206 (P:N), CD44 and CD206 (N:N), or CD181 and CD354 (N:P).

The combination of two types of molecules of regDC markers is even more preferably, for example, a combination of CD172a and CD200R (N:P), CD172a and CD44 (N:N), or CD200R and CD44 (P:N).

The combination of two types of molecules of regDC markers is still even more preferably, for example, a combination of CD200R and CD44 (P:N).

In this specification, the detection, selection, and identification do not refer to detection, selection, and identification only specific to regulatory dendritic cells. In this specification, the detection, selection, and identification also allow for simultaneously detecting, selecting, and identifying other types of cells having a regDC marker, as well.

In the present invention, the phrase "a molecule is present" means that the molecule can be detected by a method used to detect a cell, and the phrase "a molecule is absent" means that the molecule cannot be detected with the detection sensitivity of a method used to detect a cell.

Examples of methods for detecting and separating cells in which one or more types of regDC positive markers are present and/or cells in which one or more types of regDC negative markers are not present in a cell population that comprises regulatory dendritic cells include methods that use flow cytometry and mass cytometry, as well as magnetic cell separation methods. These methods can be carried out using known methods. For example, cells in which one or more types of regDC positive markers are present and/or cells in which one or more types of regDC negative markers are not present can be detected and separated by using a method that comprises a step of contacting the cells with a substance (e.g., an antibody) that specifically binds to each regDC marker. Such a substance may itself include a detectable label (e.g., GFP), or itself not include a label. When the substance itself is not labeled, the detection and separation can be achieved by further using a substance with a detectable label to directly or indirectly recognize the substance. For example, when the substance is an antibody, a fluorescent dye, a metal isotope, or a bead (e.g., a magnetic bead) can be loaded directly or indirectly onto the antibody, whereby the regDC marker on the cell surfaces can be labeled, and the cells can be detected on the basis of the labeling. The antibody here may be used singly, or in a combination of two or more types.

In the present invention, the phrases "obtaining a cell population enriched for regulatory dendritic cells" and "selecting regulatory dendritic cells" are synonymous with separating regulatory dendritic cells from a cell population that comprises regulatory dendritic cells on the basis of the regDC markers.

According to the method of the present invention, the use of the regDC markers enables detection of regulatory dendritic cells in a cell population that comprises regulatory dendritic cells, and enables enrichment of regulatory dendritic cells in a cell population that comprises regulatory dendritic cells.

The cell population according to the present invention is characterized by being enriched for regulatory dendritic cells that express or do not express one or more types of molecules selected from the group consisting of CD99, CD183, CD107a, CD366, CD172a, CD200R, CD44, CD206, CD181, CD354, CD300c, CD50, and CD84.

The cell population can be obtained, for example, by the method for producing a cell population enriched for regulatory dendritic cells of the present invention described above, or the method for enriching regulatory dendritic cells of the present invention described above.

In the present invention, the phrase "a molecule is expressed" means that the molecule can be detected by a method used for detecting cells, and the phrase "a molecule is not expressed" means that the molecule cannot be detected based on the detection sensitivity of a method used for detecting cells.

In the present invention, a cell population "enriched for regulatory dendritic cells" means that the percentage of regulatory dendritic cells that express one or more types of the regDC positive markers and/or does not express one or more types of the regDC negative markers (the number of such regulatory dendritic cells/the total number of cells in the cell population) is higher than that in conventional cell populations. In particular, the percentage of the regulatory dendritic cells is preferably 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100%.

The cell population according to the present invention, which is enriched for regulatory dendritic cells, has excellent immune tolerance-inducing effects.

The reagent for detecting regulatory dendritic cells according to the present invention is characterized by comprising one or more antibodies that are each against each of one or more types of molecules selected from the group consisting of CD99, CD183, CD107a, CD366, CD172a, CD200R, CD44, CD206, CD181, CD354, CD300c, CD50, and CD84.

When the detection reagent according to the present invention comprises antibodies against two or more types of regDC markers, the reagent can be provided as a reagent kit containing each antibody in a separate reagent. The antibodies contained in the detection reagent according to the present invention can be provided in the form of binding to, for example, a fluorescent dye, a metal isotope, or a bead (e.g., a magnetic bead). The reagent according to the present invention can also comprise antibodies other than those mentioned above (e.g., other antibodies that are capable of detecting regulatory dendritic cells).

The reagent for detecting regulatory dendritic cells according to the present invention, which comprises an antibody against a regDC marker, can be used in the method for detecting regulatory dendritic cells of the present invention described above, the method for producing a cell population enriched for regulatory dendritic cells of the present invention described above, and the method for enriching regulatory dendritic cells of the present invention described above.

The pharmaceutical composition according to the present invention is characterized by comprising the cell population enriched for the regulatory dendritic cells described above.

The pharmaceutical composition according to the present invention, which comprises the cell population enriched for the regulatory dendritic cells, is capable of suppressing or avoiding hyperactive immune responses.

The pharmaceutical composition according to the present invention can be produced as an oral or parenteral formulation by, for example, mixing an effective amount of the cell population enriched for regulatory dendritic cells with a pharmaceutically acceptable carrier according to known means. The pharmaceutical composition according to the present invention is usually produced as a parenteral formulation, such as injections, suspensions, and intravenous solutions. Examples of pharmacologically acceptable carriers include excipients, analgesics, buffers, preservatives, stabilizers, suspending agents, isotonic agents, and surfactants.

The dosage of the pharmaceutical composition according to the present invention can be appropriately determined according to various conditions such as the patient's weight, age, gender, and symptoms. The pharmaceutical composition according to the present invention is applicable to mammals including humans.

The pharmaceutical composition according to the present invention is useful in the treatment and/or prevention of organ transplant rejection, autoimmune diseases (e.g., Crohn's disease, ulcerative colitis, celiac disease, rheumatoid arthritis, systemic lupus erythematosus, psoriasis, multiple sclerosis, amyotrophic lateral sclerosis, narcolepsy, and Parkinson's disease), allergic diseases (e.g., pollinosis, food allergy, drug allergy, asthma, atopic dermatitis, eczema, food hypersensitivity, urticaria, allergic rhinitis, and allergic conjunctivitis), and graft-versus-host diseases.

### Examples

The present invention is described in further detail below with reference to Examples. However, the present invention is not limited to the Examples etc.

### Example 1: Study with the use of Ff-I01s04

As a cell population that comprised hematopoietic progenitor cells (HPCs), a floating cell population in which iPS cells (Ff-I01s04) were differentiated according to known methods (e.g., the methods disclosed in Cell Reports, 2(2012), 1722-1735 and WO2017/221975) was used. Specifically, Ff-I01s04 was seeded at 3×10⁵ cells/well in an ultra-low adhesion-treated 6-well plate (Day 0). The cells were cultured under hypoxic conditions (5% 0₂) for 5 days in EB medium (StemPro34 supplemented with 10 ug/ml human insulin, 5.5 ug/ml human transferrin, 5 ng/ml sodium selenite, 2 mM L-glutamine, 45 mM α-monothioglycerol, and 50 ug/ml ascorbic acid) supplemented with 10 ng/ml BMP4, 5 ng/ml bFGF, 15 ng/ml VEGF, and 2 pM SB431542 (Day 5). Subsequently, 50 ng/ml SCF, 30 ng/ml TPO, and 10 ng/ml Flt3L were added to perform culture for another 5 to 9 days (up to Day 14) to obtain a floating cell population. The medium was changed every 2 or 3 days during the culture period.

Next, as a cell population that comprised regulatory dendritic cells (regDCs), a cell population in which the HPCs obtained above were differentiated according to known methods (e.g., Transplantation 84: 196-206, 2007, Gene Therapy 18: 874-883, 2011, Scientific Reports 4: 3979, 2014) was used. Specifically, HPCs were seeded at 1×10⁵ cells/well in a 6-well plate, and cultured for 11 days in DC medium (a 1:1 mixture of Macrophage-SFM and CTS OpTmizer T-Cell Expansion SFM) supplemented with 20 ng/ml GM-CSF and 20 ng/ml M-CSF (Day 14 to Day 25) to obtain myeloid progenitor cells. The medium was changed every 3 or 4 days during the culture period. The obtained myeloid progenitor cells were seeded at 1×10⁶ cells/well in a 6-well plate, and cultured for 7 to 8 days in DC medium supplemented with 20 ng/ml GM-CSF, 20 ng/ml IL-4, 20 ng/ml IL-10, and 20 nM Vitamin D3 (Day 25 to Day 32 or 33) to obtain a cell population that comprised regDCs. The cytokines were added twice during the culture period (Day 27 and Day 28). To identify IL-10-producing cells in the cell population that comprised regDCs, staining was performed using the antibody sets shown in Table 1.

The cells were dispensed into a 96-well V-bottom plate, and centrifuged at 300 g for 5 minutes at room temperature. After the supernatant was removed, 100 µl of a mixture of an antibody against a surface molecule and cell staining buffer (autoMACS Rinsing Solution, Miltenyi Biotec) was added, and the resulting product was allowed to stand at 4°C for 20 minutes. Subsequently, 150 µl of cell staining buffer was added, and centrifugation was performed at 300 g for 5 minutes at room temperature. The supernatant was removed, 100 µl of cell fixation liquid (fixation buffer, Biolegend) was added, and the resulting product was allowed to stand for 15 minutes at room temperature. Thereafter, 100 µl of intracellular staining buffer (Intracellular Staining Permeabilization Wash Buffer, Biolegend) was added, and centrifugation was performed at 300 g for 5 minutes at room temperature. The supernatant was removed, 100 µl of a mixture of IL-10 antibody and intracellular staining buffer was added, and the resulting product was allowed to stand for 20 minutes at room temperature. Then, 150 µl of intracellular staining buffer was added, and centrifugation was performed at 300 g for 5 minutes at room temperature. The supernatant was removed, 200 µl of cell staining buffer was added, and the resulting product was transferred to a 5-ml tube for analysis on a flow cytometer. Data were acquired on a FACS LSR Fortessa X20 flow cytometer, and analyzed using FlowJo software.

**Table 1**

| Antibody set | Antibody | Manufacturer | Fluorescent molecule binding to antibody |
|---|---|---|---|
| 1 | CD11b | Biolegend | APC-Cy7 |
| | CD11c | Biolegend | PE-Cy7 |
| | CD183 | Biolegend | APC |
| | CD107a | Biolegend | BV421 |
| | CD99 | Biolegend | FITC |
| | CD366 | Biolegend | BV510 |
| | IL-10 | Biolegend | PE |
| 2 | CD11b | Biolegend | APC-Cy7 |
| | CD11c | Biolegend | PE-Cy7 |
| | CD200R | Biolegend | APC |
| | CD44 | Biolegend | BV421 |
| | CD172a | Biolegend | FITC |
| | CD206 | Biolegend | BV510 |
| | IL-10 | Biolegend | PE |
| 3 | CD11b | Biolegend | APC-Cy7 |
| | CD11c | Biolegend | PE-Cy7 |
| | CD354 | Biolegend | APC |
| | CD181 | Biolegend | FITC |
| | IL-10 | Bioleqend | PE |

The distribution of expression of each surface marker on IL-10-producing cells (regDCs) in DC marker (CD11b and CD11c)-positive cells in the cell population that comprised regDCs was analyzed. Specifically, the regDC-containing cell population was fractionated into four fractions (negative + positive, positive + positive, positive + negative, and negative + negative) according to two markers in each antibody set, and the percentage of IL-10-producing cells contained in the fractions was determined. Tables 2 to 4 below show the candidate markers, the percentage of IL-10-producing cells in each fraction (the percentage of IL-10-producing cells contained in each fraction), the percentage of IL-10-producing cells present in each fraction (the percentage of cells that express or do not express each marker from among IL-10-producing cells in each fraction), and the fractionation percentage. Tables 2 to 4 respectively show the results in terms of the antibody sets 1 to 3.

For the antibody sets 1 to 3, the IL-10 production percentages in the entire fractions were 3.82%, 4.06%, and 1.58%, respectively. Specifically, when the percentage of IL-10 production in a fraction was greater than that of the entire fraction, IL-10-producing cells (regDCs) were considered to be enriched. Further, the greater the percentage of IL-10-producing cells present in each fraction, the more accurate detection of IL-10-producing cells (regDCs) was possible.

**Table 2**

| Cell fraction | Percentage of IL-10-producing cells in fraction (%) | Percentage of IL-10-producing cells present in each fraction (%) | Fraction percentage (%) |
|---|---|---|---|
| CD99 positive | 0 | 0 | 0.577 |
| CD99 negative | 3.84 | 99.9 | 99.4 |
| CD183 positive | 5.6 | 73.7 | 50.522 |
| CD183 negative | 2.0 | 26.2 | 49.455 |
| CD107a positive | 0.7 | 15.7 | 84.81 |
| CD107a negative | 21.2 | 84.4 | 15.2 |
| CD366 positive | 3.0 | 64.6 | 81.5 |
| CD366 negative | 7.3 | 35.5 | 18.6 |
| CD99 negative, CD183 positive | 5.6 | 73.7 | 50.1 |
| CD99 positive, CD183 positive | 0 | 0 | 0.422 |
| CD99 positive, CD183 negative | 0 | 0 | 0.155 |
| CD99 negative, CD183 negative | 2.0 | 26.2 | 49.3 |
| CD99 negative, CD107a positive | 0.7 | 15.7 | 83.9 |
| CD99 positive, CD107a positive | 0 | 0 | 0.91 |
| CD99 positive, CD107a negative | 0 | 0 | 0.2 |
| CD99 negative, CD107a negative | 21.5 | 84.4 | 15 |
| CD99 negative, CD366 positive | 3.7 | 58.2 | 60.7 |
| CD99 positive, CD366 positive | 1.2 | 6.4 | 20.8 |
| CD99 positive, CD366 negative | 0.7 | 0.6 | 3 |
| CD99 negative, CD366 negative | 8.6 | 35.0 | 15.6 |
| CD183 negative, CD107a positive | 0.1 | 0.6 | 29.8 |
| CD183 positive, CD107a positive | 0.7 | 9.3 | 50 |
| CD183 positive, CD107a negative | 23.5 | 79.4 | 12.9 |
| CD183 negative, CD107a negative | 5.5 | 10.5 | 7.21 |
| CD183 negative, CD366 positive | 0.3 | 2.3 | 32.8 |
| CD183 positive, CD366 positive | 4.1 | 45.3 | 42.3 |
| CD183 positive, CD366 negative | 9.0 | 37.1 | 15.7 |
| CD183 negative, CD366 negative | 6.3 | 15.1 | 9.17 |
| CD107a negative, CD366 positive | 22.8 | 44.2 | 7.41 |
| CD107a positive, CD366 positive | 0.5 | 8.7 | 69.5 |
| CD107a positive, CD366 negative | 0 | 0 | 3.88 |
| CD107a negative, CD366 negative | 9.3 | 46.9 | 19.2 |

**Table 3**

| Cell fraction | Percentage of IL-10-producing cells in fraction (%) | Percentage of IL-10-producing cells present in each fraction (%) | Fraction percentage (%) |
|---|---|---|---|
| CD172a positive | 2.8 | 3.3 | 4.653 |
| CD172a negative | 4.1 | 96.7 | 95.37 |
| CD200R positive | 60.0 | 95.1 | 6.433 |
| CD200R negative | 0.2 | 4.9 | 93.59 |
| CD44 positive | 0.3 | 1.6 | 22.12 |
| CD44 negative | 5.1 | 98.3 | 77.91 |
| CD206 positive | 1.2 | 9.8 | 32.22 |
| CD206 negative | 5.4 | 90.2 | 67.82 |
| CD172a negative, CD200R positive | 61.4 | 91.8 | 6.07 |
| CD172a positive, CD200R positive | 36.4 | 3.3 | 0.363 |
| CD172a positive, CD200R negative | 0 | 0 | 4.29 |
| CD172a negative, CD200R negative | 0.2 | 4.9 | 89.3 |
| CD172a negative, CD44 positive | 0.3 | 1.6 | 20.7 |
| CD172a positive, CD44 positive | 0 | 0 | 1.42 |
| CD172a positive, CD44 negative | 3.1 | 1.6 | 2.11 |
| CD172a negative, CD44 negative | 5.2 | 96.7 | 75.8 |
| CD172a negative, CD206 positive | 1.3 | 9.8 | 30.7 |
| CD172a positive, CD206 positive | 0 | 0 | 1.52 |
| CD172a positive, CD206 negative | 3.9 | 4.1 | 4.22 |
| CD172a negative, CD206 negative | 5.5 | 86.2 | 63.6 |
| CD200R negative, CD44 positive | 0 | 0 | 18 |
| CD200R positive, CD44 positive | 2.4 | 1.6 | 2.74 |
| CD200R positive, CD44 negative | 75.2 | 98.4 | 5.31 |
| CD200R negative, CD44 negative | 0 | 0 | 74 |
| CD200R negative, CD206 positive | 0 | 0 | 36.7 |
| CD200R positive, CD206 positive | 16.4 | 17.0 | 4.22 |
| CD200R positive, CD206 negative | 87.9 | 82.9 | 3.83 |
| CD200R negative, CD206 negative | 0 | 0 | 55.3 |
| CD44 negative, CD206 positive | 3.5 | 20.3 | 23.5 |
| CD44 positive, CD206 positive | 0.5 | 2.4 | 20.5 |
| CD44 positive, CD206 negative | 0 | 0 | 6.63 |
| CD44 negative, CD206 negative | 6.4 | 77.2 | 49.3 |

**Table 4**

| Cell fraction | Percentage of IL-10-producing cells in fraction (%) | Percentage of IL-10-producing cells present in each fraction (%) | Fraction percentage (%) |
|---|---|---|---|
| CD181 positive | 0.6 | 12.5 | 35.09 |
| CD181 negative | 2.1 | 87.4 | 64.83 |
| CD354 positive | 14.3 | 91.5 | 10.12 |
| CD354 negative | 0.1 | 8.3 | 89.8 |
| CD181 negative, CD354 positive | 18.7 | 83.2 | 7.03 |
| CD181 positive, CD354 positive | 4.3 | 8.3 | 3.09 |
| CD181 positive, CD354 negative | 0.2 | 4.2 | 32 |
| CD181 negative, CD354 negative | 0.1 | 4.2 | 57.8 |

### Example 2: Study with the use of TKT3V1-7

A cell population obtained by subjecting iPS cells (TKT3V1-7) to the method described in Example 1 was used as a cell population that comprised regulatory dendritic cells. The regDC-containing cell population was stained with the antibody sets shown in Table 5 below.

**Table 5**

| Antibody set | Antibody | Manufacturer | Fluorescent molecule binding to antibody |
|---|---|---|---|
| 4 | CD11b | Biolegend | APC-Cy7 |
| | CD11c | Biolegend | PE-Cy7 |
| | CD200R | Biolegend | APC |
| | CD44 | Biolegend | BV421 |
| | CD99 | Biolegend | FITC |
| | IL-10 | Biolegend | PE |
| 5 | CD11b | Biolegend | APC-Cy7 |
| | CD11c | Biolegend | PE-Cy7 |
| | CD183 | Biolegend | APC |
| | CD44 | Biolegend | BV421 |
| | CD99 | Biolegend | FITC |
| | IL-10 | Biolegend | PE |
| 6 | CD11b | Biolegend | APC-Cy7 |
| | CD11c | Biolegend | PE-Cy7 |
| | CD200R | Biolegend | APC |
| | CD107a | Biolegend | BV421 |
| | CD99 | Biolegend | FITC |
| | IL-10 | Biolegend | PE |
| 7 | CD11b | Biolegend | APC-Cy7 |
| | CD11c | Biolegend | PE-Cy7 |
| | IL-10 | Biolegend | APC |
| | CD44 | Biolegend | BV421 |
| | CD99 | Biolegend | FITC |
| | CD50 | Biolegend | PerCP-Cv5.5 |
| | CD300c | Biolegend | PE |
| 8 | CD11b | Biolegend | APC-Cy7 |
| | CD11c | Biolegend | PE-Cy7 |
| | IL-10 | Biolegend | APC |
| | CD44 | Biolegend | BV421 |
| | CD99 | Biolegend | FITC |
| | CD50 | Biolegend | PerCP-Cv5.5 |
| | CD84 | Bioleqend | PE |

An analysis was conducted in the same manner as in Example 1. Tables 6 to 10 below show the marker candidates, the percentage of IL-10-producing cells in each fraction, the percentage of IL-10-producing cells present in each fraction, and the fraction percentage. Tables 6 to 10 respectively show the results in terms of the antibody sets 4 to 8.

For the antibody sets 4 to 8, the percentages of IL-10 production in the entire fractions were 1.77%, 2.01%, 2.3%, 3.48%, and 3.56%, respectively.

**Table 6**

| Cell fraction | Percentage of IL-10-producing cells in fraction (%) | Percentage of IL-10-producing cells present in each fraction (%) | Fraction percentage (%) |
|---|---|---|---|
| CD99 positive | 8.2 | 7.3 | 1.575 |
| CD99 negative | 1.7 | 92.3 | 98.4 |
| CD200R positive | 12.8 | 96.0 | 13.31 |
| CD200R negative | 0.1 | 3.7 | 86.665 |
| CD44 positive | 3.2 | 11.9 | 6.642 |
| CD44 negative | 1.7 | 88.0 | 93.332 |
| CD99 negative, CD200R positive | 13.3 | 88.7 | 11.8 |
| CD99 positive, CD200R positive | 8.6 | 7.3 | 1.51 |
| CD99 positive, CD200R negative | 0 | 0 | 0.065 |
| CD99 negative, CD200R negative | 0.1 | 3.7 | 86.6 |
| CD99 negative, CD44 positive | 2.5 | 8.2 | 5.96 |
| CD99 positive, CD44 positive | 9.5 | 3.7 | 0.682 |
| CD99 positive, CD44 negative | 0 | 0 | 0.032 |
| CD99 negative, CD44 negative | 1.7 | 88.0 | 93.3 |
| CD200R negative, CD44 positive | 0 | 0 | 8.7 |
| CD200R positive, CD44 positive | 2.9 | 12.8 | 7.87 |
| CD200R positive, CD44 negative | 28.0 | 82.7 | 5.23 |
| CD200R negative, CD44 negative | 0.1 | 4.6 | 78.2 |

**Table 7**

| Cell fraction | Percentage of IL-10-producing cells in fraction (%) | Percentage of IL-10-producing cells present in each fraction (%) | Fraction percentage (%) |
|---|---|---|---|
| CD99 positive | 4.7 | 45.3 | 19.55 |
| CD99 negative | 1.4 | 55.1 | 80.42 |
| CD183 positive | 2.1 | 90.8 | 87.6 |
| CD183 negative | 1.6 | 9.6 | 12.37 |
| CD44 positive | 6.0 | 20.9 | 7.03 |
| CD44 negative | 1.7 | 79.4 | 92.995 |
| CD99 negative, CD183 positive | 1.5 | 55.1 | 72.8 |
| CD99 positive, CD183 positive | 4.9 | 35.7 | 14.8 |
| CD99 positive, CD183 negative | 4.1 | 9.6 | 4.75 |
| CD99 negative, CD183 negative | 0 | 0 | 7.62 |
| CD99 negative, CD44 positive | 3.2 | 7.0 | 4.33 |
| CD99 positive, CD44 positive | 10.4 | 14.0 | 2.7 |
| CD99 positive, CD44 negative | 8.8 | 2.6 | 0.595 |
| CD99 negative, CD44 negative | 1.7 | 76.8 | 92.4 |
| CD183 negative, CD44 positive | 3.5 | 10.5 | 5.99 |
| CD183 positive, CD44 positive | 14.4 | 11.3 | 1.58 |
| CD183 positive, CD44 negative | 1.8 | 76.5 | 84.5 |
| CD183 negative, CD44 negative | 0.4 | 1.7 | 7.95 |

**Table 8**

| Cell fraction | Percentage of IL-10-producing cells in fraction (%) | Percentage of IL-10-producing cells present in each fraction (%) | Fraction percentage (%) |
|---|---|---|---|
| CD99 positive | 23.1 | 11.6 | 1.15 |
| CD99 negative | 2.1 | 88.4 | 98.8 |
| CD200R positive | 10.7 | 88.4 | 18.95 |
| CD200R negative | 0.3 | 11.6 | 81 |
| CD107a positive | 1.6 | 65.5 | 92.8 |
| CD107a negative | 11.0 | 34.6 | 7.22 |
| CD99 negative, CD200R positive | 9.9 | 76.8 | 17.8 |
| CD99 positive, CD200R positive | 23.1 | 11.6 | 1.15 |
| CD99 positive, CD200R negative | 0 | 0 | 0 |
| CD99 negative, CD200R negative | 0.3 | 11.6 | 81 |
| CD99 negative, CD107a positive | 0.7 | 25.0 | 81 |
| CD99 positive, CD107a positive | 7.9 | 40.5 | 11.8 |
| CD99 positive, CD107a negative | 12.0 | 17.3 | 3.32 |
| CD99 negative, CD107a negative | 10.2 | 17.3 | 3.9 |
| CD200R negative, CD107a positive | 0.4 | 13.4 | 81.2 |
| CD200R positive, CD107a positive | 9.1 | 38.5 | 9.78 |
| CD200R positive, CD107a negative | 16.8 | 46.2 | 6.33 |
| CD200R negative, CD107a negative | 1.6 | 1.9 | 2.7 |

**Table 9**

| Cell fraction | Percentage of IL-10-producing cells in fraction (%) | Percentage of IL-10-producing cells present in each fraction (%) | Fraction percentage (%) |
|---|---|---|---|
| CD99 positive | 17.4 | 35.4 | 7.09 |
| CD99 negative | 2.4 | 64.4 | 92.9 |
| CD300c positive | 1.0 | 22.2 | 80.26 |
| CD300c negative | 13.7 | 77.6 | 19.73 |
| CD44 positive | 16.9 | 35.8 | 7.4 |
| CD44 negative | 2.4 | 64.2 | 92.6 |
| CD50 positive | 98.7 | 66.5 | 2.345 |
| CD50 negative | 1.2 | 33.3 | 97.64 |
| CD99 negative, CD300c positive | 0.6 | 13.6 | 77.9 |
| CD99 positive, CD300c positive | 12.7 | 8.6 | 2.36 |
| CD99 positive, CD300c negative | 19.7 | 26.8 | 4.73 |
| CD99 negative, CD300c negative | 11.8 | 50.9 | 15 |
| CD99 negative, CD44 positive | 14.6 | 24.8 | 5.9 |
| CD99 positive, CD44 positive | 25.7 | 11.1 | 1.5 |
| CD99 positive, CD44 negative | 14.3 | 0.4 | 0.1 |
| CD99 negative, CD44 negative | 2.4 | 63.8 | 92.5 |
| CD99 negative, CD50 positive | 99.0 | 42.7 | 1.5 |
| CD99 positive, CD50 positive | 98.3 | 23.9 | 0.845 |
| CD99 positive, CD50 negative | 6.4 | 11.5 | 6.24 |
| CD99 negative, CD50 negative | 0.8 | 21.8 | 91.4 |
| CD300c negative, CD44 positive | 17.3 | 33.0 | 6.63 |
| CD300c positive, CD44 positive | 15.1 | 5.8 | 1.33 |
| CD300c positive, CD44 negative | 1.3 | 30.9 | 86 |
| CD300c negative, CD44 negative | 17.7 | 30.5 | 6 |
| CD300c negative, CD50 positive | 96.8 | 50.1 | 1.8 |
| CD300c positive, CD50 positive | 84.5 | 24.8 | 1.02 |
| CD300c positive, CD50 negative | 0.2 | 4.1 | 83.6 |
| CD300c negative, CD50 negative | 5.4 | 20.9 | 13.5 |
| CD44 negative, CD50 positive | 97.3 | 44.7 | 1.6 |
| CD44 positive, CD50 positive | 100.0 | 23.4 | 0.816 |
| CD44 positive, CD50 negative | 7.4 | 15.2 | 7.13 |
| CD44 negative, CD50 negative | 0.6 | 16.4 | 90.4 |

**Table 10**

| Cell fraction | Percentage of IL-10-producing cells in fraction (%) | Percentage of IL-10-producing cells present in each fraction (%) | Fraction percentage (%) |
|---|---|---|---|
| CD99 positive | 11.7 | 0.9 | 0.286 |
| CD99 negative | 3.5 | 99.0 | 99.69 |
| CD84 positive | 17.1 | 45.1 | 8.142 |
| CD84 negative | 2.4 | 60.9 | 91.834 |
| CD44 positive | 10.3 | 23.6 | 8.189 |
| CD44 negative | 3.0 | 76.4 | 91.817 |
| CD50 positive | 99.3 | 72.7 | 2.604 |
| CD50 negative | 1.0 | 27.3 | 97.353 |
| CD99 negative, CD84 positive | 17.2 | 38.1 | 7.89 |
| CD99 positive, CD84 positive | 13.3 | 0.9 | 0.252 |
| CD99 positive, CD84 negative | 0 | 0 | 0.034 |
| CD99 negative, CD84 negative | 2.4 | 60.9 | 91.8 |
| CD99 negative, CD44 positive | 9.9 | 21.7 | 7.77 |
| CD99 positive, CD44 positive | 16.0 | 1.9 | 0.419 |
| CD99 positive, CD44 negative | 28.6 | 0.9 | 0.117 |
| CD99 negative, CD44 negative | 2.9 | 75.5 | 91.7 |
| CD99 negative, CD50 positive | 99.3 | 70.3 | 2.52 |
| CD99 positive, CD50 positive | 100.0 | 2.4 | 0.084 |
| CD99 positive, CD50 negative | 3.7 | 0.5 | 0.453 |
| CD99 negative, CD50 negative | 1.0 | 26.9 | 96.9 |
| CD84 negative, CD44 positive | 0 | 0 | 1.19 |
| CD84 positive, CD44 positive | 12.2 | 26.5 | 7.72 |
| CD84 positive, CD44 negative | 42.6 | 55.6 | 4.65 |
| CD84 negative, CD44 negative | 0.7 | 17.9 | 86.4 |
| CD84 negative, CD50 positive | 69.9 | 68.9 | 3.51 |
| CD84 positive, CD50 positive | 89.3 | 23.6 | 0.94 |
| CD84 positive, CD50 negative | 2.2 | 3.8 | 6.24 |
| CD84 negative, CD50 negative | 0.2 | 3.8 | 89.3 |
| CD44 negative, CD50 positive | 95.9 | 66.5 | 2.47 |
| CD44 positive, CD50 positive | 100.0 | 17.4 | 0.621 |
| CD44 positive, CD50 negative | 4.0 | 11.4 | 10.1 |
| CD44 negative, CD50 negative | 0.2 | 4.7 | 86.9 |

## Claims

1. A method for detecting regulatory dendritic cells, the method comprising:
step a: detecting the presence or absence of one or more types of molecules on a cell surface in a cell population that comprises regulatory dendritic cells; and
step b: identifying regulatory dendritic cells on the basis of the presence or absence of the one or more types of molecules.

2. The method according to claim 1, wherein the one or more types of molecules are selected from the group consisting of CD99, CD183, CD107a, CD366, CD172a, CD200R, CD44, CD206, CD181, CD354, CD300c, CD50, and CD84.

3. The method according to claim 1, wherein the one or more types of molecules are two or more types of molecules selected from the group consisting of CD99, CD183, CD107a, CD366, CD172a, CD200R, CD44, CD206, CD181, CD354, CD300c, CD50, and CD84.

4. The method according to any one of claims 1 to 3, wherein the cell population that comprises regulatory dendritic cells is a cell population obtained by inducing differentiation of pluripotent stem cells.

5. The method according to claim 4, wherein the pluripotent stem cells are human-induced pluripotent stem cells.

6. A method for producing a cell population enriched for regulatory dendritic cells, the method comprising:
step 1: detecting the presence or absence of one or more types of molecules on a cell surface in a cell population that comprises regulatory dendritic cells; and
step 2: obtaining a cell population enriched for regulatory dendritic cells on the basis of the presence or absence of the one or more types of molecules.

7. The method according to claim 6, wherein the one or more types of molecules are selected from the group consisting of CD99, CD183, CD107a, CD366, CD172a, CD200R, CD44, CD206, CD181, CD354, CD300c, CD50, and CD84.

8. The method according to claim 6, wherein the one or more types of molecules are two or more types of molecules selected from the group consisting of CD99, CD183, CD107a, CD366, CD172a, CD200R, CD44, CD206, CD181, CD354, CD300c, CD50, and CD84.

9. A method for enriching regulatory dendritic cells, the method comprising:
step A: detecting the presence or absence of one or more types of molecules on a cell surface in a cell population that comprises regulatory dendritic cells; and
step B: selecting regulatory dendritic cells on the basis of the presence or absence of the one or more types of molecules.

10. A cell population enriched for regulatory dendritic cells obtained by the method of any one of claims 6 to 9.

11. A cell population enriched for regulatory dendritic cells that express or do not express one or more types of molecules selected from the group consisting of CD99, CD183, CD107a, CD366, CD172a, CD200R, CD44, CD206, CD181, CD354, CD300c, CD50, and CD84.

12. A cell population enriched for regulatory dendritic cells that express or do not express two or more types of molecules selected from the group consisting of CD99, CD183, CD107a, CD366, CD172a, CD200R, CD44, CD206, CD181, CD354, CD300c, CD50, and CD84.

13. A reagent for detecting regulatory dendritic cells, the reagent comprising one or more antibodies that are each against each of one or more types of molecules selected from the group consisting of CD99, CD183, CD107a, CD366, CD172a, CD200R, CD44, CD206, CD181, CD354, CD300c, CD50, and CD84.

14. A pharmaceutical composition comprising the cell population enriched for regulatory dendritic cells of any one of claims 10 to 12.
